# EUROPEAN PATENT SPECIFICATION

(11) **EP 2 606 820 B1**
(45) Date of publication and mention of the grant of the patent: **30.10.2019**
(21) Application number: 11194276.9
(22) Date of filing: 19.12.2011
(51) Int. Cl.: A61B 5/083, A61B 5/097

(54) **Airway adapter and analyzer and method for analyzing at least one property of a respiratory gas**
Luftwegadapter und Analysegerät sowie Verfahren zur Analyse von mindestens einer Eigenschaft eines Atemgases
Adaptateur de conduites d'air et analyseur et procédé pour analyser au moins une propriété de gaz respiratoires

(43) Date of publication of application: 26.06.2013
(73) Proprietor: General Electric Company, Schenectady, NY 12345 (US)
(72) Inventor: Haveri, Heikki Antti Mikael, 03150 Huhmar (FI)
(74) Representative: Fennell, Gareth Charles

(56) References cited:
- EP-A1- 2 179 691
- EP-A2- 1 661 514
- WO-A1-2011/054117
- US-A- 5 932 877
- US-A1- 2002 098 120
- US-A1- 2007 032 818
- US-A1- 2007 225 612

## Description

### BACKGROUND OF THE INVENTION

This disclosure relates generally to an airway adapter including a sampling chamber for allowing a respiratory gas flow. The airway adapter also includes a first port for delivering respiratory gas to the sampling chamber and a second port for removing respiratory gas from the sampling chamber. The disclosure also relates to an analyzer and a method for analyzing at least one property of a respiratory gas.

Tidal volume (TV) is an amount of an air inspired or taken into the lungs in a single breath. TV is also dependent on the sex, size, height, age and a health etc. of a patient. In general TV also decreases as the size of the patient decreases. In an average healthy adult, TV is about 400-600 ml whereas in an average healthy neonate, that measures 3.5-4 kg and is 50 cm tall, TV is approximately 25-50 ml. On the other hand, in an average premature neonate that measures only 500 grams TV is only about 2-3.5 ml. TV of a smaller patient is very difficult to measure, but it can be approximated to 4-7 ml/kg, applying a general rule of thumb for approximating the TV of the human lung. In practice the TV of the patient suffering pulmonary system deficiency is normally less than the approximation gives.

When the patient is mechanically ventilated with a conventional ventilator, an endotracheal tube is placed into a trachea so that it goes through oral or nasal cavity and larynx. In tracheostomy endotracheal tube goes straight into trachea through neck. The other end of the endotracheal tube is connected to a breathing circuit Y-piece through a luer type connector. If the patient is gas monitored with a mainstream or sidestream gas analyzer, an airway adapter used for sampling the breathing gas that is analyzed by the gas analyzer is normally connected between connectors of the endotracheal tube and the breathing circuit Y-piece. During an inspiration the fresh breathing gas including higher oxygen (O₂) concentration flows into the patients lungs through an inspiratory limb of the breathing circuit Y-piece, the airway adapter, the endotracheal tube and their connectors, then to a trachea, a bronchus, a bronchi, bronchioles and finally reaching an alveoli deep in the lungs, where all the gas exchange actually occurs. Carbon dioxide (CO₂) molecules in hemoglobin of a blood flowing in tiny blood vessels around the alveoli are replaced with O₂ molecules in the fresh breathing gas through the thin walls of the alveoli. O₂ molecules take their place in the hemoglobin, whereas CO₂ molecules flow out from the patient within the used expired breathing gas, through the same path as the fresh gas came in during the inspiration. Thus a gas concentration of the breathing gas measured by the gas analyzer is somewhat proportional to the gas concentration in the blood.

A volume in a space between a connection of the inspiratory and expiratory limbs of the Y-piece and the patient's mouth or nose, a beginning of oral and nasal cavities, is called a mechanical dead volume or dead space, whereas the volume in a space between patient's mouth or nose and the entrance of alveoli is called an anatomical dead volume. The part of the lung that is injured or damaged for some reason and does not participate for the gas exchange is called more specific a physical dead volume. It is obvious that as the used breathing gas flows out from the patient's lungs through the expiratory limb during expiration, a part of the used gas never exits a pulmonary system, as well as the patient side of the breathing circuit, but remains in the mechanical and anatomical dead volume. Then as the fresh gas is inspired in to the lungs through the inspiratory limb the used gas already in the anatomical and mechanical dead volume flows into the lungs before the fresh gas. The used gas fills up some or all of the alveoli depending on a ratio of the dead volume and TV or at least mixes up with the fresh gas decreasing the concentration of O₂ as well as increasing the concentration of CO₂ in the lungs, which in turn decreases the gas exchange in the alveoli. This means that the larger the dead space, the larger the volume of the used gas, with a low O₂ and high CO₂ concentration, that flows back to the patients lungs during the inspiration and worse the gas exchange in the alveoli. In other words, if the total dead volume were larger than TV or as large as TV, the patient would not get any fresh gas into the lungs, but respires the used gas back and forth in the dead volume. In practice a diffusion of gases assists the gas exchange over the dead volume little, especially when there is some movement of gases such as high frequency ventilation evolved, but the overall gas exchange in the alveoli would be lethal or dangerously poor anyway.

The anatomical dead volume is almost impossible to reduce, but it is proportional to the size and the physical condition of the patient. The mechanical dead volume depends on a breathing circuit design, defined by the inner diameter of the tubing, connectors and additional accessories, which is something that patient is not used to suffer from and can be considered as abnormal situation. Additional accessories are usually different type of breathing circuit connectors such as a so-called peep saver, T-connector, L-connector etc. used to build up a suitable circuit for the treatment of the patient and to add devices such as gas analyzers, humidifiers, nebulizers etc. to the circuit. These additional accessories and devices increase the mechanical dead volume considerably. Obviously the mechanical dead volume is more critical for smaller patients with smaller TV or patients suffering barotraumas etc., which also decrease TV and for this reason many devices cannot be used for the treatment of smaller patients.

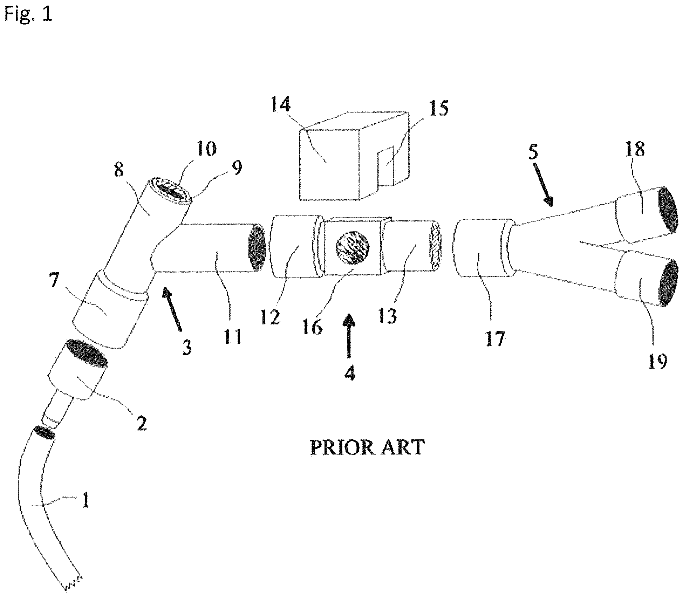

The conventional patient side part of the breathing circuit, which is also shown in Figure 1, usually consists of an endotracheal tube 1, male type luer connector 2, so-called PEEP-saver 3 (PEEP = Positive End Expiratory Pressure) and breathing circuit Y-piece 5 that connects the patient side part of the breathing circuit to the ventilator through breathing circuit tubing (not shown). If sidestream or mainstream gas analyzing is desired to measure inspiratory and expiratory breathing gas concentration the airway adapter 4 needs to be connected between PEEP-saver 3 and Y-piece 5.

The other end of endotracheal tube 1 enters the patient's trachea (not shown) and the other end comprises a male type luer connector 2. The connector 2 is oftentimes firmly attached to the end of endotracheal tube 1. The conventional, so-called PEEP-saver 3 comprises three tubular openings. The first opening 7, which is a female type luer connector, can be connected to the male type luer connector 2 at the end of endotracheal tube 1. The second opening 8 straight at the opposite side of the first opening 7 comprises an elastic part 9 with a thin membrane 10 in the middle, made of material such as rubber that can be pierced with a catheter. The catheter is used to suck mucus, blood and body fluids from the patient's lungs to keep the lungs open for the gas exchange. The third opening 11 is a male type luer connector that is adjacent to and in sharp angle relative to the second opening 8. The conventional airway adapter 4 (mainstream type in this case) includes two tubular ports. The first port 12 of airway adapter 4, which is a female type luer connector, can be connected to the male type third opening 11 of the PEEP-saver 3 or the male type luer connector 2 at the end of endotracheal tube 1. A second port 13 of the airway adapter 4 is a male type luer connector. The conventional Y-piece 5 comprises three tubular apertures as well. A first aperture 17, which is a female type luer connector, can be connected to the male type luer second port 13 of airway adapter 4, the male type luer third opening 11 of the PEEP-saver 3 or the male type luer connector 2 at the end of endotracheal tube 1. An inspiratory limb 18 of the Y-piece 5 connects to a ventilator (not shown in Figure 1) through an inspiratory tubing to carry inspiratory fresh gas through the circuit into the patient whereas an expiratory limb 19 connects to the ventilator through an expiratory tubing to carry expiratory gas out from the patient. To enable gas concentration analyzing with this particular mainstream type gas sensor body 14 needs to be placed on airway adapter 4 so that cavity 15 in mainstream sensor 14 encloses the sampling cell 16 of airway adapter 4.

The inner diameter of endotracheal tube 1 can vary from 2 mm to 10 mm or more or in terms of a cross-sectional area approximately from 3 to 79 mm² or more and the length can vary from 150mm to 250 mm or more depending on the patient it is connected to. In general the inner diameter (ID) of the endotracheal tube 1 increases as the age (or the size proportional to the age) of the patient increases. In general the smaller the patient the smaller the endotracheal tube 1 used. Table 1 below shows some recommendations for the use of endotracheal tubes with different aged patients from manufacturers.

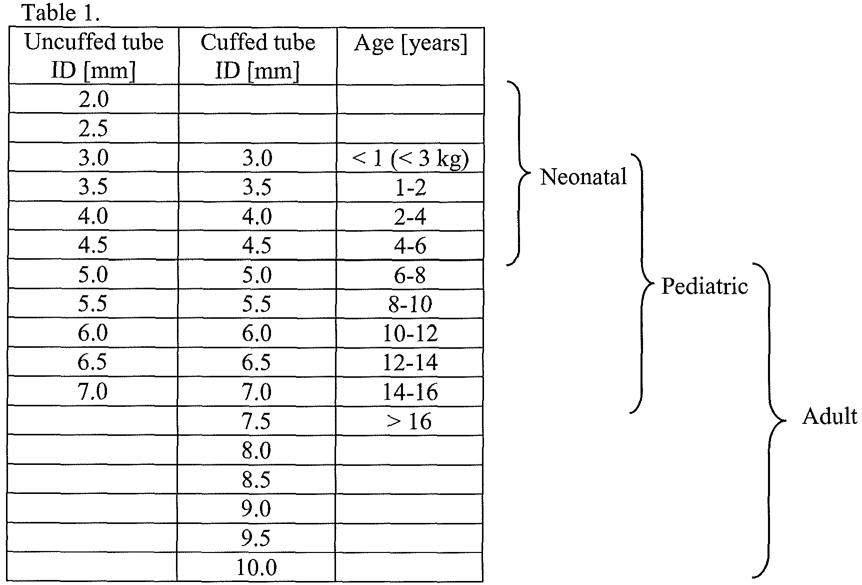

Connectors 2 are conventionally used to connect very different size of the endotracheal tubes 1 to one size of the PEEP-saver 3, airway adapter 4 or Y-piece 5, which means that each size of endotracheal tube 1 needs a separate connector 2 connected to it. Other end of the connector 2 is a standard size male connector that fits in to the female first opening 7 of the PEEP-saver 3, female first port 12 of airway adapter 4 or female first aperture 17 of Y-piece and the other end is tubular connector that fits to its respective endotracheal tube. The total length of the connector 2 is approximately 31 mm, the length of the tubular part approximately 9 mm and the length of the male connector approximately 22 mm.

All of the male and the female type luer connectors in the circuit are standard size and all the males and the females connect to each other regardless of which connectors or pieces are connected together. Although all the pieces and connectors can be placed freely into different locations in the circuit the PEEP-saver 3 should be connected always to the connector 2 of the endotracheal tube 1 to ensure that the catheter has the shortest and free entrance into the patient's lungs. The free placement of different parts thus causes a disadvantage for misplacing the parts.

The PEEP-saver is continuously used at least with pediatric and adult patients and the lungs are usually sucked empty of body fluids with a catheter through the PEEP-saver many times per day at least in ICU (intensive care unit). Although its advantage of saving the pressure and the flow inside the circuit during the treatment the disadvantage is the large dead volume, approximately 10-15 ml, it adds to the circuit. Similarly the connector 2 of the endotracheal tube 1 adds a dead volume of approximately 1.5 ml alone and when it is connected to one of the female first opening 7, first port 12 and first aperture 17 the dead volume of the connection increases up to 2-3 ml. This is approximately the increase in dead volume of each male-female connection. The dead volume of the whole breathing circuit shown in Figure 1 may be as high as 25 ml.

Conventional airway adapters are relatively big in size and their dead volume is large, approximately 3-6 ml with male first port 12 and female second port 13. The cavity for breathing gases to flow through the adapter is very un-tubular comprising stepped changes, corners and transversal cavities. Furthermore as the airway adapter is connected to the rest of the breathing circuit, as shown in Figure 1, the overall dead volume of the breathing circuit becomes considerably large, up to tens of milliliters. On breathing circuit system level this is one of the reasons that gas analyzing cannot be used for smaller patients. This is easy to understand if the conventional breathing circuit system with conventional airway adapter is connected to the patient that weights 1 kg through the endotracheal tube with the inner diameter of 2.5 mm is at best more than 7 ml. This is at least two times or rather many times higher than TV of the patient in this example, which is approximately 4-7 ml. This means that the patient in the above example rebreaths the used gas and is more likely to suffer from the poor gas exchange than to get better treatment

Also the number of male and female connections, each causing step like change into the breathing circuit flow path, as well as large volumes of each connector and piece, cause turbulences into the gas flow that in turn mix and dilute the fresh gas flowing towards the patient and the used gas flowing out from the patient decreasing the gas exchange in the lungs.

Although the conventional mainstream gas analyzers are able to measure gas concentrations at higher RR more than 60 breaths/minute with high TV such as 400-600 ml, the overall breathing circuit dead volume degrades the gas concentration measurement at least with smaller patients since the inspiratory and expiratory gases mix in the volume where the airway adapter is located also. Furthermore the breathing circuit as well as the airway adapter comprises many stepped changes in the breathing gas flow path formed by multiple connections between different accessories, as shown in Figure 1 and explained in paragraphs 6 and 7. These stepped changes and corners generate turbulences that mix up and dilute the barrier between inspiratory and expiratory gases thus distorting the measured gas concentration and capnogram formed from the gas concentration values in time. It is obvious that performance of this kind of system, as also shown in Figure 1 and described in paragraphs 6 and 7, degrades rapidly as RR increases and TV decreases, thus it is not suitable for measuring smaller patients.

Thus at the moment there does not exist a proper breathing gas concentration analyzing technique for smaller patients. The high overall dead volume together with non-existing breathing gas analyzing are also reasons why conventional ventilation cannot be used in many cases or at least it is difficult or even dangerous to use. Due to the weaknesses of conventional ventilation patients are more likely ventilated with high frequency ventilators (HFV) with RR up to 3000. These ventilators do not have the conventional inspiration and expiration phase as normal respiration, but the gas exchange in the alveoli is ensured through the diffusion of gases. HFV has its own drawbacks in addition that the gas diffusion type high frequency ventilation also makes it impossible to measure breathing gas concentrations comparable to the gas concentration in the alveoli with any conventional gas analyzer technology. The high frequency ventilation does not have the normal inspiration and expiration phase, but is more like a vibration and the diffusion of the gases, which makes it impossible to gas monitor the patient with the conventional gas analyzing techniques. Moreover the high frequency ventilators are noise and their functionality disputed. The only way to analyze the gas exchange in the lungs is to measure CO₂ and O₂ concentration from the blood through blood samples or a transcutaneous measurement. The blood sampling is very stressful and even dangerous for the small patient whose blood volume is very small. The transcutaneous measurement has its own weaknesses, such as a need for connections to the patient's skin so that oxygen in the blood just under the skin can be measured. Especially the skin of premature neonates is very thin and fragile and thus the measurement is not very often used. Connections also come loose from the patient easily and the technique is such that it heats up the patient at the connections, which place thus has to be changed. The blood sampling is not a real time measurement, as it has to be analyzed in the laboratory, which in turn causes a long time delay into an acute patient care. As there is no real time measurement to analyze the gas exchange of small patients, they are usually ventilated insufficiently, which causes different trauma for the patient and a longer time to recover.

WO 2011/054117 discloses a pressure measuring system comprising a sensor assembly, and an airway adapter with a fluid chamber. The sensor assembly comprises a compartment in pressure connection with the fluid chamber, a mechanically operated actuator for enabling and disabling pressure transmission across a measurement port and a pressure sensor for measuring pressure in the compartment. There is a membrane between the compartment and the fluid chamber to separate a medium in the compartment from medium in the fluid chamber.

US 5932877 discloses a side stream infrared gas analyzer for detecting concentration of a gaseous component of a flow through an airway adapter. The analyzer comprises an infrared energy detector, a sample cell receiving gas flow stream, a source of infrared energy emitting energy through the sample cell for detection by the detector.

EP 2179691 discloses an airway adapter comprising a sampling chamber for allowing a respiratory gas flow and a sensor integrated into the airway adapter. The sensor includes an electrolyte in contact with the respiratory gas for analyzing the gas.

US2002/0098120 discloses an airway adapter for an optical sensor comprising a memory.

### BRIEF DESCRIPTION OF THE INVENTION

The present invention is defined in the accompanying claims.

The above-mentioned shortcomings, disadvantages and problems are addressed herein which will be understood by reading and understanding the following specification.

In an embodiment, an airway adapter includes a sampling chamber for allowing a respiratory gas flow, a first port for delivering respiratory gas to the sampling chamber, and a second port for removing respiratory gas from the sampling chamber. The airway adapter also includes a memory adapted to store information relevant to at least one fact of the airway adapter having an influence on its usability.

In another embodiment, an analyzer for analyzing at least one property of a respiratory gas includes an airway adapter having a sampling chamber adapted to allow the respiratory gas flow, a first port for delivering respiratory gas to the sampling chamber, and a second port for removing respiratory gas from the sampling chamber. The analyzer for analyzing at least one property of a respiratory gas also includes a sensor adapted to acquire a signal indicative of at least one property of the respiratory gas flowing through the sampling chamber of the airway adapter, and a processing unit adapted to process the signal indicative of at least one property of the respiratory gas. The airway adapter also includes a memory adapted to store information relevant to at least one fact of the airway adapter having an influence on its usability.

In yet another embodiment, a method for analyzing at least one property of a respiratory gas includes allowing the respiratory gas flow through an airway adapter, and acquiring a signal indicative of at least one a property of the respiratory gas. The method for analyzing at least one property of a respiratory gas also includes processing the signal indicative of at least one a property of the respiratory gas and reading an information stored in the airway adapter relevant to at least one fact of the airway adapter having an influence on its usability.

Various other features, objects, and advantages of the invention will be made apparent to those skilled in art from the accompanying drawings and detailed description thereof.

### BRIEF DESCRIPTION OF THE DRAWINGS

Figure 1 shows an exploded view of a prior art breathing circuit;
Figure 2 shows a schematic view of a breathing circuit incorporating an analyzer in accordance with an embodiment;
Figure 3 shows an exploded perspective, schematic view of an analyzer incorporating a sensor and an airway adapter seen from obliquely upward on the front side, but which analyzer represents a wireless embodiment in accordance with another embodiment;
Figure 4 shows a perspective, schematic view of an analyzer in Figure 3 in running order;
Figure 5 shows an exploded perspective, schematic view of an analyzer incorporating a sensor and an airway adapter in Figure 3 seen from obliquely downward on the back side;
Figure 6 shows a perspective, schematic view of an airway adapter in accordance with another embodiment; and
Figure 7 shows an exploded perspective, schematic view of an airway adapter detached from a branching unit in accordance with another embodiment.

### DETAILED DESCRIPTION OF THE INVENTION

Specific embodiments are explained in the following detailed description making a reference to accompanying drawings. These detailed embodiments can naturally be modified and should not limit the scope of the invention as set for in the claims.

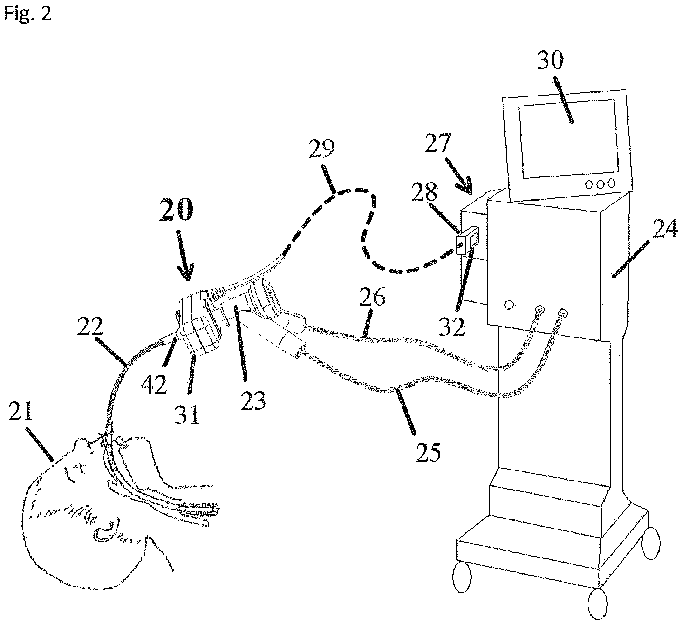

Figure 2 shows a breathing circuit incorporating an analyzer 20 such as a new mainstream type breathing gas analyzer for analyzing at least one property of a respiratory gas connected to an intubated patient 21 through an endotracheal tube 22 and also connected to a branching unit 23, such as Y-piece, that further connects to a ventilator 24 through an inspiratory tube 25 that allows fresh gas flow into the patient lungs and an expiratory tube 26 to allow used gas or an expiratory gas to flow out from the patient's lungs. The analyzer 20 comprising a sensor 31 and an airway adapter 42 can also be connected electrically to a host device 27, which is in this case incorporates also a ventilator 24, but can also be such as patient monitor, anesthesia machine, transportable monitor, hand held device or similar. The host device in the embodiment shown in Figure 2 is able to transfer electric power from the host device 27 into a connector 28 and through this connector 28 and an electric cable 29 into the analyzer 20 to operate the sensor 31. The host device 27 or connector 28 may comprise a processing unit 32, such as a central processing unit CPU, or it can be located into the sensor itself to operate the sensor 31 and measurements to calculate gas concentration values, pressures, flows etc. Vice versa the measurement information, such as breathing gas concentration, flow, pressure or similar can be transferred from the analyzer 20 to the host device 27 to show the information for the care giver or user on a display 30 or similar.

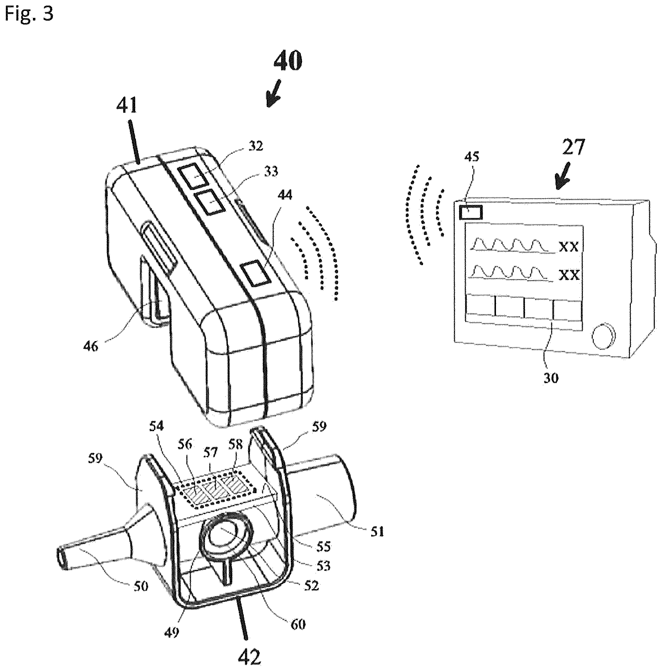

Figure 3 shows an exploded view of the analyzer 40 in accordance with another embodiment with a main exception to the analyzer 20 in Figure 2 that there is a wireless connection between the analyzer and the host device 27. The sensor 41 connects to the airway adapter 42. The airway adapter 42 can be identical in both embodiment shown in Figures 2 and 3. The sensor 41 can acquire a signal indicative of at least one property of a respiratory gas and the processing unit 32, such as CPU, within the sensor as shown in Figure 3 or outside the sensor as shown in Figure 2 can process this signal and, if desired, determine at least one property of the respiratory gas. Inside the sensor 41 there may be a battery 33, especially in the wireless embodiment in Figure 3, to operate the analyzer 40, but the embodiment of Figure 2 does not necessarily need the battery because of the cable 29 as explained hereinbefore. The analyzer 40 connects wirelessly to the host device 27, for example a patient monitor as shown in Figure 3, through radio frequency transceiver 44 or similar wireless method or technology located inside the sensor 41 so that the measurement information, such as breathing gas concentration, flow, pressure or similar can be transmitted from the sensor 41 to host device's 27 transceiver 45 to be used by host device and to display the information for the care giver or user. The analyzer 20 or its sensor 31 shown in Figure 2 provides the measurement information along the cable 29 to the processing unit 32 which can be displayed, too. The host device 27 in Figure 3 can be a ventilator, anesthesia machine, hand held device or similar as well or the display can also be integrated on one of the sensor 41 surfaces (not shown in Figures) to show information at the analyzer.

Some hospitals and users prefer disposable airway adapters, but also other breathing circuit accessory made disposable to reduce contamination risk. Patient's lungs generate mucus and other secretions and injured lungs bleed blood, which easily enter the breathing circuit accessory forming a good environment for bacteria and viruses to survive and reproduce. To minimize the contamination risk a disposable accessory need to be changed and reusable accessory to be cleaned frequently enough.

The airway adapter 42 comprises a sampling chamber 49 allowing a respiratory gas flow through this chamber. Also the airway adapter comprises a first port 50 for delivering respiratory gas to the sampling chamber that can be connected to the patient airways for instance directly in case the adapter is in a mask or the adapter is part of the mask attachable to patient's face or that can be connected through the endotracheal tube 22 to patient airways. The airway adapter further comprises a second port 51 for removing the respiratory gas from the sampling chamber and for instance operationally connecting with the branching unit 23 as shown in Figure 2. Further the airway adapter may comprise at least one component 60 for enabling measurement of the respiratory gas property such as a gas compound or concentration of the respiratory gas, pressure and/or flow etc. The component of the gas measuring equipment may be for example at least one optical window 52, but typically there may be two optical windows, one on both sides of the sampling chamber, for allowing infrared radiation to penetrate the respiratory gas inside the sampling chamber forming a measurement path. Typically the measurement path is between two opposite optical windows leaving therebetween the sampling chamber with the respiratory gas.

Further the airway adapter comprises a memory 54 for storing information relevant to at least one fact of the airway adapter having an influence on its usability. The fact influencing on the usability may be for instance a patient related fact or a technical fact. Technical facts are for instance a feature or property of the adapter, a shape of the sampling chamber, a time the adapter has been in use, an age of the at least one component, estimated useful life time, an optical property of the component 60 such as the optical window 52, a material of the component, a length of the measurement path and/or an identification information, such as a number etc. Patient related facts may be for instance a size of the airway adapter, inner diameter of the intubation tube connected to that or the size of the sampling chamber and patient size specific information, which is proportional to physical size of the airway adapter, such as use ranges for RR, TV, breathing circuit peak pressure, positive end expiratory pressure, inspired and expired gas concentrations, and other values derived from previous such as energy expenditure. Also alarm limits for all measured values, waveform sweep speeds and amplitudes etc. that can be automatically adjusted for each size of patient to reduce false alarms. It is also possible to communicate patient related facts to other devices such as nebulizers, which are used to deliver medicine into the patients lungs, to ensure for example correct patient size specific dosing of medicine thus to avoid under or over dosing automatically. Thus patient related facts can be used, if desired, to control other devices affecting to the patient.

The memory, such as EEPROM or similar for storing data, may be in or on a wall 53 of the airway adapter 42 or the sampling chamber 49, advantageously integrated inside the wall 53. On a surface 55 of the wall 53 there may be first contacts 56, 57 and 58 to enable electrical operation of the memory 54. These first contacts may also be part of the memory. The airway adapter 42 connects with the sensor 31 or 41 when the airway adapter slides into a cavity 46 so that the surface 55 of airway adapter is towards the sensor 31 or 41 and when the airway adapter 42 reaches the bottom of cavity 46 the sensor 31 or 41 locks between wings 59.

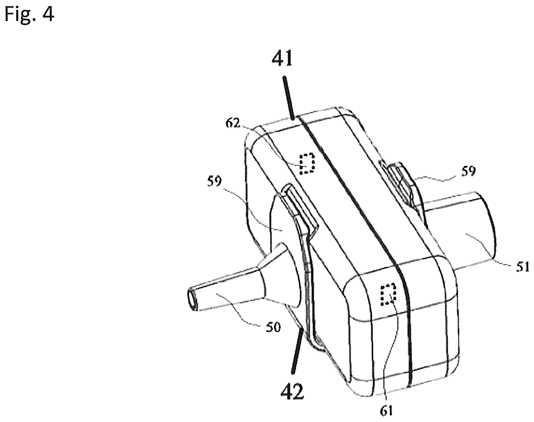

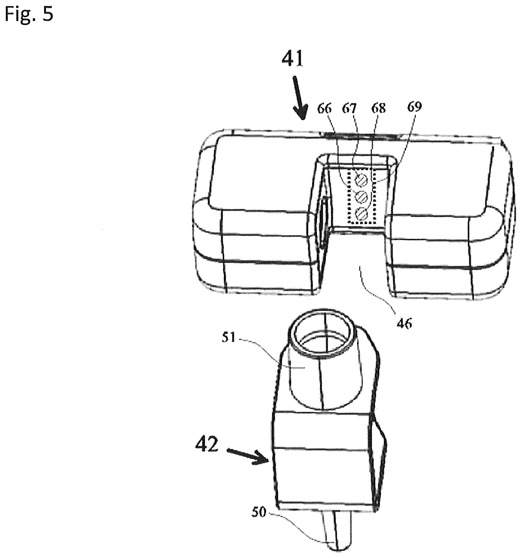

Figure 4 shows another view when the airway adapter 42 placed into sensor 41, but as well this Figure 4 embodiment may be identical with the analyzer 20 and its sensor 31 and airway adapter 42. When the airway adapter 42 is placed into the sensor 41, the sampling chamber 49 and the respiratory gas flowing through are exposed to a radiation, such as infrared radiation, coming from a radiation source 61, such as an infrared radiation source, located inside the sensor 41 in line with the component 60 which is in this embodiment the at least one optical window 52 of the airway adapter 42 so that radiation can traverse through the optical window 52, shown in Figure 3, through the sampling chamber 49 and another optical window (not shown in Figures) into a detector 62, such as an infrared detector, located on the other side of the airway adapter 42 inside the sensor 41. When the airway adapter 42 is placed into the sensor 41 the first contacts 56, 57 and 58 on the surface 55 of airway adapter 42 touch corresponding second contacts 66, 67 and 68 in a connector 69 in the bottom of the cavity 46 of the sensor 41 for providing an electrical connection as shown in Figure 5. Through these first contacts 56, 57 and 58 in airway adapter 42 and the second contacts 66, 67 and 68 in the sensor 41 the sensor can electrically operate the memory 54 in the airway adapter 42. Naturally the gas analysis can be based on any other principle than the infrared technology.

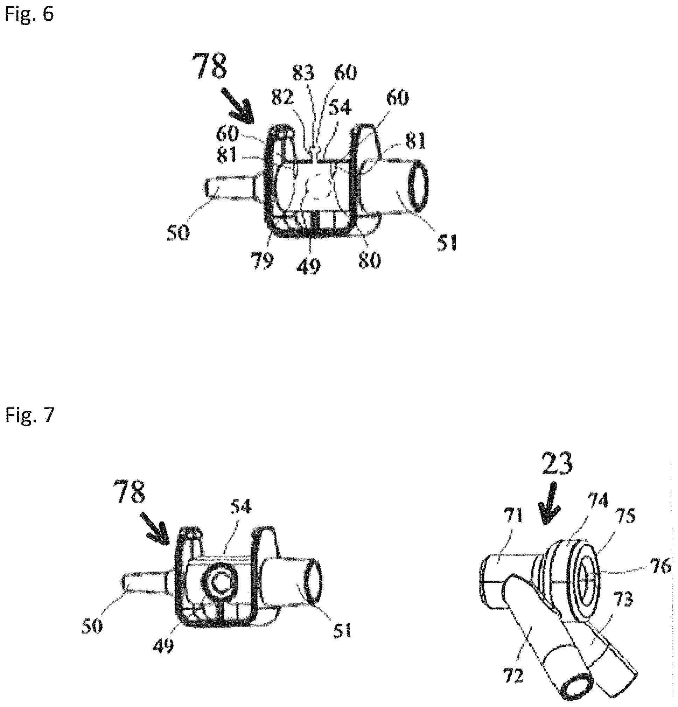

Airway adapter 78 shown in the schematic view in Figure 6 may also comprise a flow measurement device 81 for measuring breathing gas flow and a pressure measurement device 83 for measuring breathing gas pressure. Both the flow measurement device 81 and the pressure measurement device 83 are components 60 for enabling measurement of the respiratory gas property. In this particular embodiment shown in Figure 6 the flow component of breathings gas can be measured with at least one thermistor, but more advantageously with two thermistors 79 and 80 which are typically at the end of protrusions inside the sampling chamber 49 to be able to determine the direction of flow as well. Thermistors 79 and 80 may be for example hot wires, placed between the first port 50 and the second port 51. There are many different methods to measure the gas flow based on especially two thermistors, thus those are not explained here. The airway adapter 42 may also comprise a third port 82 to interconnect pressure measurement device 83 to the sampling chamber 49 between the first port 50 and the second port 51 to allow breathing gas pressure measurement. The airway adapter may also comprise a fourth port (not shown in Figure 6) beside the third port along the breathing gas flow path to interconnect another pressure sensor (not shown in Figure 6) to the sampling chamber 49 between the first port 50 and the second port 51 and a flow barrier (not shown in Figure 6) between the third and fourth ports to allow flow measurement based on pressure difference over the flow barrier between the third and fourth port. The airway adapter 78 also comprises the memory 54 and first contacts 56, 57, 58 as explained earlier and hereafter.

The airway adapter can also be understood as nasal prongs, nasal mask, nasal and oral mask or face mask, which comprises the sampling chamber 49 or other similar device for analyzing at least one breathing gas component. These airway adapters may be reusable or disposable and they may comprise the memory 54 and first contacts 56, 57, 58 to store data that can be used for purposes as described earlier and hereafter. When the gas sensor is attached to the airway adapter the analyzing of at least one breathing gas component is enabled as described earlier and hereafter.

It is obvious that data transfer between the memory 54 in the airway adapter 42 or 78 and sensor 31 or 41 can be accomplished with radio frequency identification (RFID), communication based on capacitive, inductive, magnetic, light or other similar short range data transfer technology. Suitable method depends on the size, cost and reliability of connectivity whether these techniques are suitable and useful for small, miniaturized, reusable or disposable usage. If for example RFID is used, the "RFID tag" comprising memory, supporting circuitry and antenna for short range radio frequency communication would replace EEPROM-type memory 54 inside the wall 53 of airway adapter. Similarly the connector 69, with electrical second contacts 66, 67, 68 in the cavity 46, would be replaced with the radio frequency transceiver that communicates the data, but also the electrical power between the "RFID tag" in the airway adapter 42 and radio frequency transceiver in sensor 31 or 41, when the airway adapter is placed close to or into the cavity 46. Electrical contact may be better in this particular case, as it is easy to ensure that the airway adapter 42 is correctly placed into the sensor 31 or 41 through electric properties of the electrical contact as they connect between the airway adapter 42 and the sensor 31 or 41.

Figure 7 shows the airway adapter 42 and the branching unit 23 detached from each other. The airway adapter designed for neonates is miniaturized and can be made similar between endotracheal tube sizes from 2 mm to 4.5 mm, but the diameter of the first port 50 needs to be specific for each size of endotracheal tube sizes between 2 mm and 4.5 mm to avoid additional connectors. The breathing gas sampling chamber 49 and the cavity between the first port 50 and the second port 51 is designed as tubular as possible in regard with endotracheal tube 22 and the branching unit 23 where the airway adapter 42 connects to. Tubular means that all step like changes have been minimized, the cross sectional area of the cavity between the first port 50 and the second port 51 changes as little as possible and there is no sharp edges or corners in the flow path that may generate gas turbulences.

The branching unit 23 shown in Figure 7 replaces the conventional breathing circuit Y-piece that was shown in Figure 1, even though the conventional one can be also used with the airway adapter 42. The branching unit 23 comprises at least four tubular apertures. A first aperture 71, which is a female type luer connector, can be connected to the male type luer second port 51 of airway adapter 42. An inspiratory limb 72 of the branching unit 23 connects to a ventilator 24 as shown in Figure 2 through an inspiratory tube 25 to carry inspiratory fresh gas through the circuit into the patient 21 whereas an expiratory limb 73 connects to the ventilator 24 through an expiratory tube 26 to carry expiratory gas out from the patient. The second aperture 74 straight at the opposite side of the first aperture 71 comprises an elastic part 75 with a shutter 76, such as a thin membrane, in the middle, made of a material such as rubber that can be pierced with a catheter, endoscope or nebulizer. The catheter is used to suck mucus, blood and body fluids from the patient's lungs to keep the lungs open for the gas exchange, the endoscope is used for viewing lungs and the nebulizer for administrating aerosolized medicine into the lungs. As there is no gas flow through the second aperture 74 at any time and the second aperture 74 is located into the branching point of all four apertures the second aperture 74 does not increase the dead volume. Basically the aim of the whole new airway adapter 42 and branching unit 23 design described above and shown in Figure 7 is to keep the inner diameter and the cross sectional area of the breathing gas flow path constant from endotracheal tube 22 throughout the whole patient part of the circuit until it is divided into expiratory and inspiratory flow paths in the branching unit 23 and to keep the dead volume of the circuit as low as possible.

The airway adapter can be designed for pediatrics according to same design principles as that for neonates. The overall airway adapter design is miniaturized and made similar between endotracheal tube sizes from 5 mm to 7 mm, only the diameter of the first aperture 71 should be specific for each size of endotracheal tube sizes between 5 mm and 7 mm to avoid additional connectors. Also the branching unit is similar to branching unit 23 shown in Figure 7, but it's scale is larger to fit pediatric size patients and breathing circuit tubing.

The airway adapter for adults, may be designed to be one size for all endotracheal tube diameters >7 mm. The breathing circuit dead volume is not that critical for adults as it is for smaller patients since tidal volumes of adults are much higher whereas respiration rates are much lower and it is also common that all adult size endotracheal tubes with inner diameter >7 mm comprise firmly fixed connector with fixed connector diameter of approximately 22 mm at the breathing circuit end. Thus the diameters of the first port 50 and the second port 51 may be fixed, but the breathing gas sampling chamber 49 and the cavity between the first port 50 and the second port 51 can be designed as tubular as possible. The branching unit for adults may be similar to branching unit 23 shown in Figure 7, but it's scale is larger to fit adult size patients and breathing circuit tubing.

The size and shape difference between the smallest neonate airway adapter and the largest adult airway adapter is significant. Neonate airway adapters are used to measure very small TVs and high RR, whereas adult airway adapter is used to measure high TV and low RR. Pediatric airway adapters locate between the neonate and the adult values in TV and RR. Although airway adapters have different form and shape and they function differently in regard to each other the sensor 31 or 41 remains the same whether a neonate, pediatric or adult airway adapter is attached to it. For example even the distance between the optical windows 52 of the airway adapter may vary from airway adapter to another to achieve optimal tubular path between the endotracheal tube 22 and the airway adapter 42, thus the change in distance between the optical windows change gas absorption signal of measured gas, which accuracy can be corrected in the sensor 31 based on the information stored into airway adapters' memory 54.

Airway adapters 42 can be reusable or disposable and they can be made of plastic or similar material. The used materials and the construction of the disposable accessory need to be recyclable and low cost to enable reasonable and efficient disposability. Materials such as optical windows in airway adapters may wear out and cloud easily degrading the optical signal that the detector receives, thus degrading the measurement such as the gas concentration, gas compound, pressure, flow or other similar measurement, which can be compensated with the lifetime information stored into the memory, because it has an influence on the usability of the airway adapter 42. To ensure that airway adapters are not used too long to cause risk for the patient safety the airway adapter's time of use can be written into the memory 54 by the sensor 40, which is one fact of the airway adapter having an influence on its usability. When the time of use approaches or exceeds the estimated life time limit of particular airway adapter the sensor 31 or 41 alarms or sends an alarm signal to the host device 27 that alarms the user. Materials used in reusable airway adapters may endure longer than that of disposable airway adapters. However, for example the optical windows 52 will wear out and cloud finally degrading the measurement causing that airway adapter needs to be changed to a new one to avoid the use of too used airway adapters, but that depends on the manner and how frequently they are cleaned. To overcome patient safety problems again the time of use information can be written into the memory 54, as described for disposable airway adapters. If the airway adapter 42 is disconnected from the sensor 31 or 41 and used later the sensor 31 or 41 reads the memory 54 and starts to update the usage information. When the limit for usage is reached the sensor 31 or 41 informs the user or the host device 27 that further informs or alarms. However as the life time of reusable airway adapters depend on how and how often they are cleaned the life time may vary a lot. To optimize the life time of the airway adapter, but also to ensure patient safety issues, the sensor 31 or 41 can measure optical properties of optical windows 52 for example signal transmission through the sampling chamber 49 for the particular airway adapter with the detector(s) 62 inside the sensor. When the airway adapter 42 is placed into the sensor for the first time the signal value from the detector is written into the airway adapter's memory 54. Later on, always when that particular airway adapter is placed into any sensor, the instant value measured from the detector is compared to the value stored into the memory. The optical properties of the airway adapter change along the time it is used and cleaned and when the instant value is not within the acceptable limits (limits may be stored into the airway adapter's memory as well) in regard to the first value stored in the memory the user is alarmed to change the airway adapter.

The optical properties of optical windows may vary depending on the gas(es) to be measured having an influence on the usability of the airway adapter 42 and which fact can be stored into the memory 54. For example the optical bandwidth for measuring CO₂ is a lot different to that of inhalation anesthetics, such as desflurane. It is common to use for example sapphire as an optical window material for CO₂ measurement used commonly in ICUs, whereas for example Calcium fluoride is used for measuring anesthetics, CO₂ and N₂O in surgery. Optical signal transmissions of these two materials are different thus otherwise similar airway adapters that are placed into one type sensor need to be calibrated before the use. This problem can be solved by writing the airway adapter 42 or the window specific calibration values into the memory 54 in the airway adapter 42 that the sensor 31 or 41 uses when the particular airway adapter is placed into the sensor.

Different size of patients need size specific airway adapters that connect to size specific branching units to minimize breathing circuit dead volume causing rebreathing of gases, to make airway adapters more tubular to improve gas concentration, flow, pressure or similar measurement accuracy by reducing step like changes in the breathing gas flow path that mix and dilute gases and to make measurement more sensible and accurate in regard with respiration rate (RR). Table 3 shows the minimum and maximum values of dead volumes for neonatal and pediatric systems and dead volume for the one size of adult system.

**Table 3.**

| **Patient** | **tube ID** | **Total dead volume of airway adapter and branching unit [ml]** |
|---|---|---|
| **Neo (min)** | **2** | 0.39 |
| **Neo (max)** | **4.5** | 0.49 |
| **Pedi (min)** | **5** | 0.66 |
| **Pedi (max)** | **7** | 0.93 |
| **Adult** | **>7** | 4 |

The size of the airway adapter, which is proportional to patient's physical size and physiology, is one fact having an influence on the usability of the airway adapter 42 and can be stored into the memory 54 of every size of airway adapters. The memory may have a calibration information specific to each size of airway adapter to get the good measurement performance out of the gas concentration, pressure, flow or any other similar measurement property. The airway adapter size information stored into the airway adapter's memory can be used to adjust the performance limits, alarm limits, waveforms or similar properties at the host device 27, such as patient monitor, ventilator, hand held device or similar. This means that the sensor 31 or 41 reads the airway adapter specific information stored into airway adapters memory 54 and transmits the information through electric cable 29, radio frequency transceiver 44 or similar way into the processing unit 32 of the host device 27. The host device automatically adjusts host properties specific to the size of a patient, such as waveform or capnogram sweep speed, signal amplitude, digit fields, alarm limits and similar properties according to information it received from the sensor. For example RR or TV of a neonate is much different to that of an adult, thus for example alarm limits for RR or TV needs to be changed to save care givers time in adjusting monitoring devices and to avoid false alarms or even care malpractice due to false or missed alarms. To optimize the functioning of measurement for example the RR and TV or gas concentration measurement algorithms may be different for a different size of patients and thus also for different size of airway adapters that can be taken into use based on the information stored into the memory. Also the waveform (capnogram) sweep speed (the number of breaths) on the screen vary a lot depending on the RR (size) of a patient. Airway adapter size specific calibration, but also other size related information can be stored into the memory already at the factory.

Airway adapter memory 54 can comprise an identification information, which may be a number or similar as well used for detecting that the airway adapter has been manufactured to meet at least one of the quality and design specifications for the product to avoid bad quality of care that may lead to patient safety issues. This is also the fact having an influence on the usability of the airway adapter.

The written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. An airway adapter (42) adapted to form an analyser (40) together with a sensor (41) which sensor is arranged to measure at least one property of a respiratory gas, the airway adapter comprising:
a sampling chamber (49) for allowing a respiratory gas flow, the sampling chamber having at least two optical windows (52);
a first port (50) for delivering respiratory gas to said sampling chamber; and
a second port (51) for removing respiratory gas from said sampling chamber; and
further comprising a memory (54) adapted to store information relevant to at least one fact of said airway adapter having an influence on its usability,
**characterised in that** the memory (54) is arranged to store time of use information and optical properties of the at least two optical windows in response to a signal from the sensor (41), and **in that**
the memory (54) is further arranged to store information regarding distance between the at least two optical windows to allow correction of a gas absorption signal of measured gas.

2. The airway adapter according to claim 1 further comprising at least one component (60) for enabling measurement of a respiratory gas property.

3. The airway adapter according to claim 2, **characterized in that** the at least one component is one of at least one optical window (52) for allowing radiation to penetrate the respiratory gas inside said sampling chamber forming a measurement path, a flow measurement device (81) for measuring breathing gas flow and a pressure measurement device (83) for measuring breathing gas pressure.

4. The airway adapter according to any preceding claim, **characterized in that** the fact is one of a feature of said airway adapter, a shape of said sampling chamber, a time said airway adapter has been in use, an estimated useful life time of said airway adapter, an age of said at least one component, an optical property of said component (60), a material of said at least one component of said airway adapter, a length of said measurement path and an identification information of said airway adapter.

5. The airway adapter according to any preceding claim, **characterized in that** the fact is at least one of a size of one of said airway adapter, an inner diameter of an intubation tube connected to said airway adapter, a size of said sampling chamber and patient size specific information, which is proportional to physical size of said airway adapter, and that patient related facts can be used, if desired, to control other devices affecting to the patient.

6. The airway adapter according to claim 2, further comprising first contacts (56, 57, 58) adapted to be connectable to a sensor (31; 41), which sensor is adapted to acquire a signal indicative of at least one a property of the respiratory gas flowing through said sampling chamber, and which sensor comprises a connector (69) adapted to contact with said first contacts to enable electrical operation of said memory with said sensor (31;41), when said airway adapter and said sensor are connected together.

7. The airway adapter according to claim 6, **characterized in that** said first contacts are adapted to transfer one of stored information in said memory (54) relevant to at least one fact of said airway adapter having an influence on its usability from said adapter to said sensor (31; 41), and an information outside said airway adapter to said memory.

8. The airway adapter according to claim 6, **characterized in that** said at least one component (60) for enabling measurement of the respiratory gas property is adapted to be in contact with said sensor (31).

9. The airway adapter according to claim 5, **characterized in that** said memory having the patient related fact about the size of one of said airway adapter and said sampling chamber, is adapted to have a calibration information specific to each size to get the good measurement performance out of the gas concentration, pressure, flow or any other measurement property.

10. The airway adapter according to claim 9, **characterized in that** said size information stored into said memory can be used to adjust performance limits, alarm limits, waveforms or similar properties.

11. The airway adapter according to claim 4, **characterized in that** identification information is for detecting that the airway adapter has been manufactured to meet at least one of the quality and design specifications.

12. An analyzer for analyzing at least one property of a respiratory gas, the analyzer comprising:
an airway adapter (42; 78) according to any one of the preceding claims;
a sensor (31; 41) adapted to acquire a signal indicative of at least one property of the respiratory gas flowing through said sampling chamber of said airway adapter; and
a processing unit (32) adapted to process said signal indicative of at least one property of the respiratory gas.

13. A method for analyzing at least one property of a respiratory gas comprising:
allowing the respiratory gas flow through an airway adapter (42; 78), the airway adapter comprising a sampling chamber (49) for allowing a respiratory gas flow, the sampling chamber having at least two optical windows (52); a first port (50) for delivering respiratory gas to said sampling chamber; a second port (51) for removing respiratory gas from said sampling chamber; and a memory (54) arranged to store time of use information;
acquiring by means of a sensor (31; 41) a signal indicative of at least one property of the respiratory gas; and
processing said signal indicative of at least one a property of the respiratory gas by means of a processing unit (32);
reading by means of said sensor (31; 41) an information stored in said memory relevant to at least one fact of said airway adapter having an influence on its usability,
**characterised by** storing in the memory (54), by means of the sensor, time of use information and optical properties of the at least one optical window in said airway adapter, and by
retrieving from the memory (54), by means of the sensor, information regarding distance between the at least two optical windows to allow correction of a gas absorption signal of measured gas.

## Patentansprüche

1. Luftwegadapter (42), der zur Bildung eines Analysegeräts (40) ausgelegt ist, zusammen mit einem Sensor (41), wobei der Sensor zum Messen zumindest einer Eigenschaft eines Atemgases ausgelegt ist, wobei der Luftwegadapter das Folgende umfasst:
eine Probenkammer (49) zum Erlauben einer Atemgasströmung, wobei die Probenkammer zumindest zwei optische Fenster (52) aufweist;
eine erste Anschlussöffnung (50) zur Abgabe von Atemgas an die Probenkammer; und
eine zweite Anschlussöffnung (51) zur Entfernung von Atemgas aus der Probenkammer; und
ferner umfassend einen Speicher (54), der zum Speichern von Informationen ausgelegt ist, die für zumindest ein Faktum des Luftwegadapters relevant sind und einen Einfluss auf seine Nutzbarkeit haben,
**dadurch gekennzeichnet, dass** der Speicher (54) zum Speichern von Anwendungsdauerinformationen und optischen Eigenschaften der zumindest zwei optischen Fenster als Reaktion auf ein Signal vom Sensor (41) ausgelegt ist, und dass
der Speicher (54) ferner zum Speichern von Informationen bezüglich des Abstands zwischen den zumindest zwei optischen Fenstern ausgelegt ist, um eine Korrektur eines Gasabsorptionssignals gemessenen Gases zu erlauben.

2. Luftwegadapter nach Anspruch 1, ferner umfassend zumindest eine Komponente (60) zum Ermöglichen einer Messung einer Atemgaseigenschaft.

3. Luftwegadapter nach Anspruch 2, **dadurch gekennzeichnet, dass** die zumindest eine Komponente eine von zumindest einem optischen Fenster (52), damit Strahlung das Atemgas innerhalb der Probenkammer durchdringen kann und einen Messweg bildet, einer Strömungsmessvorrichtung (81) zum Messen der Atemgasströmung und einer Druckmessvorrichtung (83) zum Messen des Atemgasdrucks ist.

4. Luftwegadapter nach einem der vorhergehenden Ansprüche, **dadurch gekennzeichnet, dass** das Faktum eines von einem Merkmal des Luftwegadapters, einer Form der Probenkammer, einer Anwendungsdauer des Luftwegadapters, einer geschätzten Nutzungsdauer des Luftwegadapters, einem Alter der zumindest einen Komponente, einer optischen Eigenschaft der Komponente (60), einem Material der zumindest einen Komponente des Luftwegadapters, einer Länge des Messwegs und einer Identifikationsinformation des Luftwegadapters ist.

5. Luftwegadapter nach einem der vorhergehenden Ansprüche, **dadurch gekennzeichnet, dass** das Faktum zumindest eines von einer Größe eines des Luftwegadapters, einem Innendurchmesser eines mit dem Luftwegadapter verbundenen Intubationstubus, einer Größe der Probenkammer und für die Größe des Patienten spezifischen Informationen ist, die proportional zur physischen Größe des Luftwegadapters sind, und dass die den Patienten betreffenden Fakten nach Wunsch zur Steuerung anderer Vorrichtungen, die den Patienten betreffen, verwendet werden können.

6. Luftwegadapter nach Anspruch 2, ferner umfassend erste Kontakte (56, 57, 58), die zur Verbindung mit einem Sensor (31; 41) angepasst sind, wobei der Sensor zum Erfassen eines Signals angepasst ist, das auf zumindest eine Eigenschaft des durch die Probenkammer strömenden Atemgases hinweist, und wobei der Sensor eine Verbindungsglied (69) umfasst, das für einen Kontakt mit ersten Kontakten angepasst ist, um den elektrischen Betrieb des Speichers mit dem Sensor (31; 41) zu ermöglichen, wenn der Luftwegadapter und der Sensor miteinander verbunden sind.

7. Luftwegadapter nach Anspruch 6, **dadurch gekennzeichnet, dass** die ersten Kontakte zur Übertragung einer von im Speicher (54) gespeicherten Informationen, die für das zumindest eine Faktum des Luftwegadapters relevant sind und einen Einfluss auf die Nutzbarkeit des Adapters haben, an den Sensor (31; 41), und einer Information außerhalb des Luftwegadapters an den Speicher, ausgelegt sind.

8. Luftwegadapter nach Anspruch 6, **dadurch gekennzeichnet, dass** die zumindest eine Komponente (60) zum Ermöglichen einer Messung der Atemgaseigenschaft dazu angepasst ist, mit dem Sensor (31) in Kontakt zu sein.

9. Luftwegadapter nach Anspruch 5, **dadurch gekennzeichnet, dass** der Speicher mit dem den Patienten betreffenden Faktum bezüglich der Größe eines des Luftwegadapters und der Probenkammer dazu angepasst ist, eine für jede Größe spezifische Kalibrationsinformation aufzuweisen, um die gute Messleistung aus der Gaskonzentration, dem Druck, der Strömung oder einer beliebigen anderen Messeigenschaft zu erhalten.

10. Luftwegadapter nach Anspruch 9, **dadurch gekennzeichnet, dass** die im Speicher gespeicherte Größeninformation zum Einstellen der Leistungsgrenzen, Alarmgrenzen, Wellenformen oder ähnlichen Eigenschaften verwendet werden kann.

11. Luftwegadapter nach Anspruch 4, **dadurch gekennzeichnet, dass** die Identifikationsinformation dem Nachweis dient, dass der Luftwegadapter so hergestellt wurde, dass er zumindest eine der Qualitäts- und Konstruktionsspezifikationen erfüllt.

12. Analysegerät zum Analysieren zumindest einer Eigenschaft eines Atemgases, wobei das Analysegerät das Folgende umfasst:
einen Luftwegadapter (42; 78) nach einem der vorhergehenden Ansprüche;
einen Sensor (31; 41), der zum Erfassen eines Signals ausgelegt ist, das auf zumindest eine Eigenschaft des durch die Probenkammer des Luftwegadapters strömenden Atemgases hinweist; und
eine Verarbeitungseinheit (32), die zum Verarbeiten des auf die zumindest eine Eigenschaft des Atemgases hinweisenden Signals ausgelegt ist.

13. Verfahren zum Analysieren zumindest einer Eigenschaft eines Atemgases, umfassend:
Erlauben der Atemgasströmung durch einen Luftwegadapter (42; 78), wobei der Luftwegadapter eine Probenkammer (49) zum Erlauben einer Atemgasströmung aufweist, wobei die Probenkammer zumindest zwei optische Fenster (52) aufweist; eine erste Anschlussöffnung (50) zur Abgabe von Atemgas an die Probenkammer; eine zweite Anschlussöffnung (51) zur Entfernung von Atemgas aus der Probenkammer; und einen Speicher (54), der zum Speichern von Anwendungsdauerinformationen angeordnet ist;
Erfassen eines auf zumindest eine Eigenschaft des Atemgases hinweisenden Signals mittels eines Sensors (31; 41); und
Verarbeiten des auf zumindest eine Eigenschaft des Atemgases hinweisenden Signals mittels einer Verarbeitungseinheit (32);
Lesen einer im Speicher gespeicherten Information, die für zumindest ein Faktum des Luftwegadapters relevant ist, das einen Einfluss auf seine Nutzbarkeit hat, mittels eines Sensors (31; 41),
**gekennzeichnet durch** Speichern, im Speicher (54) mittels des Sensors, von Anwendungszeitinformationen und optischen Eigenschaften des zumindest einen optischen Fensters im Luftwegadapter, und durch
Abrufen, aus dem Speicher (54) mittels des Sensors, von Informationen bezüglich des Abstands zwischen den zumindest zwei optischen Fenstern, um eine Korrektur eines Gasabsorptionssignals gemessenen Gases zu erlauben.

## Revendications

1. Adaptateur des voies respiratoires (42) conçu pour former un analyseur (40) conjointement avec un capteur (41), lequel capteur est conçu pour mesurer au moins une propriété d'un gaz respiratoire, l'adaptateur des voies respiratoires comprenant :
une chambre d'échantillonnage (49) pour permettre un écoulement de gaz respiratoire, la chambre d'échantillonnage ayant au moins deux fenêtres optiques (52) ;
un premier orifice (50) pour fournir un gaz respiratoire à ladite chambre d'échantillonnage ; et
un second orifice (51) pour éliminer un gaz respiratoire de ladite chambre d'échantillonnage ; et
comprenant en outre une mémoire (54) conçue pour stocker des informations se rapportant à au moins un fait dudit adaptateur des voies respiratoires ayant une influence sur sa facilité d'utilisation,
**caractérisé en ce que** la mémoire (54) est conçue pour stocker des informations de temps d'utilisation et des propriétés optiques des deux, ou plus, fenêtres optiques en réponse à un signal provenant du capteur (41) et **en ce que**
la mémoire (54) est en outre conçue pour stocker des informations concernant une distance entre les deux, ou plus, fenêtres optiques pour permettre une correction d'un signal d'absorption de gaz d'un gaz mesuré.

2. Adaptateur des voies respiratoires selon la revendication 1, comprenant en outre au moins un composant (60) pour permettre une mesure d'une propriété de gaz respiratoire.

3. Adaptateur des voies respiratoires selon la revendication 2, **caractérisé en ce que** le ou les composants sont l'un parmi au moins une fenêtre optique (52) pour permettre un rayonnement pour faire pénétrer le gaz respiratoire à l'intérieur de ladite chambre d'échantillonnage formant un trajet de mesure, un dispositif de mesure d'écoulement (81) pour mesurer un écoulement de gaz respiratoire et un dispositif de mesure de pression (83) pour mesurer une pression de gaz respiratoire.

4. Adaptateur des voies respiratoires selon l'une quelconque des revendications précédentes, **caractérisé en ce que** le fait est l'un parmi une caractéristique dudit adaptateur des voies respiratoires, une forme de ladite chambre d'échantillonnage, une durée pendant laquelle ledit adaptateur des voies respiratoires a été utilisé, une durée de vie utile estimée dudit adaptateur des voies respiratoires, un âge dudit ou desdits composants, une propriété optique dudit composant (60), un matériau dudit ou desdits composants dudit adaptateur des voies respiratoires, une longueur dudit trajet de mesure et une information d'identification dudit adaptateur des voies respiratoires.

5. Adaptateur des voies respiratoires selon l'une quelconque des revendications précédentes, **caractérisé en ce que** le fait est une taille de l'un dudit adaptateur des voies respiratoires et/ou un diamètre interne d'un tube d'intubation raccordé audit adaptateur des voies respiratoires et/ou une taille de ladite chambre d'échantillonnage et/ou des informations spécifiques à la taille d'un patient, qui sont proportionnelles à la taille physique dudit adaptateur des voies respiratoires, et **en ce que** des faits liés au patient peuvent être utilisés, si on le souhaite, pour commander d'autres dispositifs ayant un effet sur le patient.

6. Adaptateur des voies respiratoires selon la revendication 2, comprenant en outre des premiers contacts (56, 57, 58) conçus pour pouvoir être raccordés à un capteur (31 ; 41), lequel capteur est conçu pour acquérir un signal indiquant au moins une propriété du gaz respiratoire s'écoulant à travers ladite chambre d'échantillonnage et lequel capteur comprend un connecteur (69) conçu pour venir en contact avec lesdits premiers contacts pour permettre une opération électrique de ladite mémoire avec ledit capteur (31 ; 41) lorsque ledit adaptateur des voies respiratoires et ledit capteur sont raccordés ensemble.

7. Adaptateur des voies respiratoires selon la revendication 6, **caractérisé en ce que** lesdits premiers contacts sont conçus pour transférer soit des informations stockées dans ladite mémoire (54) se rapportant à au moins un fait dudit adaptateur des voies respiratoires ayant une influence sur sa facilité d'utilisation depuis ledit adaptateur audit capteur (31 ; 41), soit une information à l'extérieur dudit adaptateur des voies respiratoires à ladite mémoire.

8. Adaptateur des voies respiratoires selon la revendication 6, **caractérisé en ce que** ledit ou lesdits composants (60) pour permettre une mesure de la propriété de gaz respiratoire sont conçus pour être en contact avec ledit capteur (31).

9. Adaptateur des voies respiratoires selon la revendication 5, **caractérisé en ce que** ladite mémoire comportant le fait associé au patient concernant la taille soit dudit adaptateur des voies respiratoires, soit de ladite chambre d'échantillonnage est conçue pour comporter une information d'étalonnage spécifique à chaque taille pour obtenir la bonne performance de mesure de la concentration, la pression, l'écoulement de gaz ou n'importe quelle autre propriété de mesure.

10. Adaptateur des voies respiratoires selon la revendication 9, **caractérisé en ce que** lesdites informations de taille stockées dans ladite mémoire peuvent être utilisées pour ajuster des limites de performance, des limites d'alarme, des formes d'onde ou des propriétés similaires.

11. Adaptateur des voies respiratoires selon la revendication 4, **caractérisé en ce que** des informations d'identification sont destinées à détecter que l'adaptateur des voies respiratoires a été fabriqué pour répondre à au moins l'une des spécifications de qualité et de conception.

12. Analyseur pour analyser au moins une propriété d'un gaz respiratoire, l'analyseur comprenant :
un adaptateur des voies respiratoires (42 ; 78) selon l'une quelconque des revendications précédentes ;
un capteur (31 ; 41) conçu pour acquérir un signal indiquant au moins une propriété du gaz respiratoire s'écoulant à travers ladite chambre d'échantillonnage dudit adaptateur des voies respiratoires ; et
une unité de traitement (32) conçue pour traiter ledit signal indiquant au moins une propriété du gaz respiratoire.

13. Procédé pour analyser au moins une propriété d'un gaz respiratoire consistant :
à permettre l'écoulement de gaz respiratoire à travers un adaptateur des voies respiratoires (42 ; 78), l'adaptateur des voies respiratoires comprenant une chambre d'échantillonnage (49) pour permettre un écoulement de gaz respiratoire, la chambre d'échantillonnage ayant au moins deux fenêtres optiques (52) ; un premier orifice (50) pour fournir un gaz respiratoire à ladite chambre d'échantillonnage ; un second orifice (51) pour éliminer un gaz respiratoire de ladite chambre d'échantillonnage ; et une mémoire (54) conçue pour stocker des informations de temps d'utilisation ;
à acquérir, au moyen d'un capteur (31 ; 41), un signal indiquant au moins une propriété du gaz respiratoire ; et
à traiter ledit signal indiquant au moins une propriété du gaz respiratoire au moyen d'une unité de traitement (32) ;
à lire, au moyen dudit capteur (31 ; 41) une information stockée dans ladite mémoire se rapportant à au moins un fait dudit adaptateur des voies respiratoires ayant une influence sur sa facilité d'utilisation,
**caractérisé par** le stockage, dans la mémoire (54), au moyen du capteur, d'informations de temps d'utilisation et de propriétés optiques de la ou des fenêtres optiques dans ledit adaptateur des voies respiratoires et par
la récupération, à partir de la mémoire (54), au moyen du capteur, d'informations se rapportant à une distance entre les deux, ou plus, fenêtres optiques pour permettre une correction d'un signal d'absorption de gaz d'un gaz mesuré.
